(19) Europäisches Patentamt
European Patent Office
Office européen des brevets

(11) Numéro de publication : **0 455 533 B1**

(12) # FASCICULE DE BREVET EUROPEEN

(45) Date de publication du fascicule du brevet : **15.12.93 Bulletin 93/50**

(51) Int. Cl.$^5$ : **C07C 69/54,** C07C 67/11

(21) Numéro de dépôt : **91401020.2**

(22) Date de dépôt : **17.04.91**

(54) **Nouveau procédé de fabrication du méthacrylate de benzyle et de ses dérivés halogenes ou alcoyles sur le noyau aromatique.**

(30) Priorité : **03.05.90 FR 9005580**

(43) Date de publication de la demande : **06.11.91 Bulletin 91/45**

(45) Mention de la délivrance du brevet : **15.12.93 Bulletin 93/50**

(84) Etats contractants désignés : **AT BE CH DE DK ES FR GB GR IT LI LU NL SE**

(56) Documents cités :
**SU-A- 416 345**
**US-A- 2 830 078**

(73) Titulaire : **ATOCHEM**
**4, Cours Michelet La Défense 10 Cédex 42**
**F-92091 Paris La Défense (FR)**

(72) Inventeur : **Grosius, Paul**
**11, rue Jeanne d'Arc**
**F-57540 Petite-Rosselle (FR)**
Inventeur : **Vanoye, Didier**
**9, rue Barabino**
**F-57600 Forbach (FR)**
Inventeur : **Hurtel, Patrice**
**2, chemin des Brasseurs, Résidence Foch**
**F-57500 St Avold (FR)**

EP 0 455 533 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention se rapporte à une nouvelle méthode de synthèse permettant l'obtention du méthacrylate de benzyle et de certains de ses dérivés de manière simple et avec un rendement élevé.

Le méthacrylate de benzyle et ses dérivés chlorés ou alcoylés sur le noyau aromatique sont des monomères méthacryliques largement utilisés en copolymérisation avec d'autres monomères, notamment acryliques, méthacryliques et vinylaromatiques. Il est donc important, du point de vue des utilisations en copolymérisation, que ces monomères puissent être obtenus de manière aussi simple et aussi économique que possible.

L'une des méthodes de synthèse du méthacrylate de benzyle et de ses dérivés les plus couramment utilisées consiste en la transestérification du méthacrylate de méthyle par l'alcool benzylique ou ses dérivés chlorés ou alcoylés sur le noyau aromatique. Cette réaction est toutefois rendue délicate par le fait que l'alcool benzylique est facilement oxydable en benzaldéhyde par l'oxygène de l'air nécessaire à la stabilisation du monomère dans les conditions de la réaction.

Une autre méthode de synthèse consiste à partir du chlorure de benzyle. Ainsi, le brevet soviétique n° 416 345 décrit la synthèse du méthacrylate de benzyle à partir de chlorure de benzyle et d'acide méthacrylique dans un solvant polaire aprotique tel que le diméthylformamide et en présence d'une quantité stoechiométrique d'un accepteur d'acide chlorhydrique tel qu'une amine tertiaire, à une température de 20°C à 60°C. En effectuant cette réaction à 50°C pendant 2,5 heures, le rendement atteint est de 75%. Par ailleurs, J. DVORAK dans Chem. Abstracts, 53, 2144b a décrit la synthèse du méthacrylate de benzyle à partir de chlorure de benzyle et de méthacrylate de sodium en présence d'une amine tertiaire. En utilisant 10% en moles de tributylamine, on obtient ainsi un rendement de 60% en méthacrylate de benzyle après 2,5 heures.

Le problème que la présente invention vise à résoudre consiste à procurer un procédé simple et économique pour la synthèse du méthacrylate de benzyle et de ses dérivés en évitant les inconvénients précités de la synthèse à partir de l'alcool benzylique tout en améliorant fortement les rendements des synthèses connues à partir du chlorure de benzyle.

La présente invention est basée sur la découverte que les buts recherchés peuvent être atteints en effectuant la réaction du chlorure de benzyle ou de ses dérivés halogénés ou alcoylés à température modérée, en présence d'une proportion véritablement catalytique d'amine tertiaire et de préférence en présence d'un solvant particulier tel que l'acétonitrile ou le toluène.

Ainsi, l'objet principal de la présente invention consiste en un procédé de fabrication du méthacrylate de benzyle ou de ses dérivés halogénés ou alcoylés par réaction du chlorure de benzyle ou d'un de ses dérivés halogénés ou alcoylés sur un méthacrylate de métal alcalin, caractérisé en ce que ladite réaction est effectuée en présence d'au plus 3% en moles, par rapport au chlorure de benzyle ou à un de ses dérivés, d'une amine tertiaire.

Le méthacrylate de benzyle ou ses dérivés halogénés ou alcoylés sur le noyau aromatique peuvent être représentés par la formule générale :

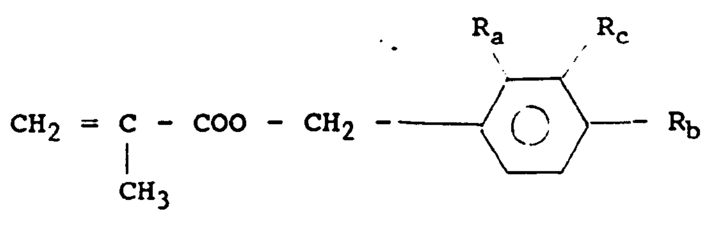

dans laquelle :

- $R_a$ = H ou Cl
- $R_b$ = H, Cl, $CH_3$ ou

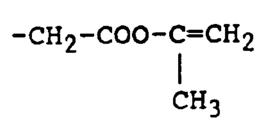

- $R_c$ = H ou $CH_2$ = CH-

Le chlorure de benzyle ou ses dérivés halogénés ou alcoylés utilisés comme produits de départ pour la mise en oeuvre du procédé de l'invention peuvent être représentés par la formule générale :

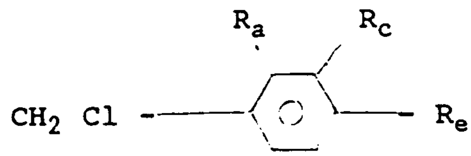

dans laquelle :

- $R_a$ = H ou Cl
- $R_e$ = H, Cl, $CH_3$ ou $CH_2Cl$,
- $R_c$ = H ou $CH_2$ = CH-

On a en effet trouvé qu'il était possible de fabriquer le méthacrylate de benzyle et ses dérivés en mettant en oeuvre le chlorure de benzyle et ses dérivés correspondants tels que le chloro-2 chlorure de benzyle, le dichloro-2,4 chlorure de benzyle, le méthyl-4 chlorure de benzyle, le chloro-méthyl-4 chlorure de benzyle, le chloro-4 chlorure de benzyle, le vinyl-3 chlorure de benzyle ...

Le méthacrylate de métal alcalin utilisé pour la réaction selon l'invention peut être un méthacrylate de lithium, de potassium ou de sodium. Lorsque le méthacrylate de potassium est utilisé, il peut être préparé par saponification du méthacrylate de méthyle ou par neutralisation de l'acide méthacrylique par le carbonate de potassium, ce dernier étant de préférence en excès par rapport à l'acide. Dans ce cas, il est possible de ne pas isoler le méthacrylate de potassium avant sa réaction avec le chlorure de benzyle ou avec un de ses dérivés et le procédé de fabrication selon l'invention peut donc être assimilé à un procédé de synthèse en deux étapes à partir de l'acide méthacrylique.

L'amine tertiaire utilisée comme catalyseur dans le procédé selon l'invention est de préférence une diamine tertiaire, par exemple de formule générale :

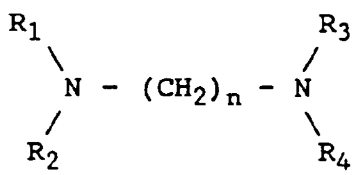

(I)

dans laquelle n est un nombre entier allant de 1 à 10, $R_1$, $R_2$, $R_3$ et $R_4$, identiques ou différents, sont des radicaux alkyle, cycloalkyle ou aryle ayant de 1 à 12 atomes de carbone, le cas échéant fonctionnalisés et le cas échéant tels qu'ils forment un hétérocycle avec l(es) atome(s) d'azote qui les portent. Des exemples de diamines tertiaires utilisables sont la tétraméthyléthylènediamine et la tétraméthylpropyldiamine. Il peut aussi s'agir d'une polyamine comportant plus de deux fonctions amine tertiaire.

Le procédé de fabrication selon l'invention est de préférence mis en oeuvre dans les conditions suivantes :

- un rapport molaire
  méthacrylate de métal alcalin/chlorure de benzyle ou ses dérivés compris entre 0,9 et 1,5 environ, de préférence entre 1,0 et 1,2 ;
- une proportion molaire
  amine tertiaire/chlorure de benzyle ou ses dérivés comprise entre 0,2% et 3% environ, de préférence entre 0,5% et 2% environ.

Le procédé de fabrication selon l'invention est effectué de préférence en présence d'un solvant de très grande polarité tel que l'acétonitrile ou le toluène qui constitue un autre solvant utilisable. D'autres solvants également utilisables sont le diméthylformamide, le tétrahydrofuranne, le diméthylsulfoxyde, la N-méthyl-pyrrolidone, le dichloro-1,2-éthane, etc. Le solvant est utilisé de préférence dans une proportion de 0,3 à 10 volumes environ pour 1 volume de chlorure de benzyle ou de ses dérivés.

Bien évidemment, la température réactionnelle sera choisie en fonction de la température d'ébullition du solvant choisi. Toutefois, en règle générale, il n'est pas nécessaire de recourir à des températures réactionnelles élevées, attendu que des températures plus modérées suffisent à garantir un rendement élevé en méthacrylate de benzyle ou en un de ses dérivés et une cinétique convenable. La température réactionnelle sera donc généralement choisie entre 20°C et 140°C environ, de préférence entre 30°C et 110°C environ. A titre d'exemple, lorsque l'acétonitrile est utilisé comme solvant, la température est choisie entre 30°C et 60°C environ.

La réaction du procédé selon l'invention est de préférence effectuée en présence d'au moins un inhibiteur de polymérisation, utilisé par exemple à raison de 0,05% à 0,5% en poids sur la base du poids de méthacrylate

de métal alcalin. Comme exemples d'inhibiteurs de polymérisation utilisables, on peut citer notamment la phénothiazine, l'éther méthylique de l'hydroquinone, la N,N-diéthyl-hydroxylamine, le nitrobenzène, le di-tertiobutylcatéchol, le butyl hydroxy toluène, l'hydroquinone, le p-anilino-phénol, le phosphite de di-(2-éthylhexyl)-octylphényle, le bleu de méthylène et leur mélanges en toutes proportions.

Le procédé de fabrication selon l'invention est de préférence mis en oeuvre sous pression atmosphérique. Toutefois, l'emploi de la pression ou d'une pression réduite n'est généralement pas nuisible aux performances de la présente invention. Le procédé peut être mis en oeuvre de manière discontinue ou continue en faisant appel aux connaissances usuelles de l'homme de l'art. La durée de la réaction est évidemment fonction des autres paramètres réactionnels déjà cités, notamment les rapports molaires des réactifs, la quantité du solvant et la température réactionnelle. Elle est toutefois généralement comprise entre 1 et 10 heures environ. A l'issue de la réaction proprement dite, le chlorure de métal alcalin formé est séparé par filtration et de préférence lavé par le même solvant que celui utilisé pour la réaction. Le filtrat est ensuite soumis à une étape d'évaporation du solvant, par exemple sous pression réduite, afin de récupérer le méthacrylate de benzyle ou le dérivé. Ceux-ci sont généralement obtenus avec une pureté au moins égale à 95%, avec une très faible quantité résiduelle de chlorure de benzyle ou du dérivé de départ. Par distillation sous pression réduite, on peut ensuite obtenir un produit de très grande pureté - supérieure à 99% - convenant aux applications précitées. L'amine tertiaire peut être récupérée en partie ou en totalité lors de la distillation ou éliminée par lavage à l'eau du produit brut avant ou après élimination du solvant. Ce dernier traitement permet également d'éliminer les traces de sel qui peuvent se révéler nuisibles lors de la purification ultérieure.

Les exemples suivants illustrent la présente invention sans en limiter la portée.

EXEMPLE 1

Dans un réacteur de 1 litre muni d'une agitation mécanique centrale et d'une colonne à distiller de 4 plateaux surmontée d'une tête de colonne avec décanteur, contenant 241 g de méthanol (7,5 moles) on introduit avec précaution 132 g de potasse à 85% (2 moles). La réaction est fortement exothermique et il faut refroidir pour maintenir la température à 60-65°C. On maintient l'agitation pendant 20 minutes jusqu'à dissolution totale. On introduit lentement dans le réacteur agité 220 g de méthacrylate de méthyle (2,2 moles), on maintient la température à 60°C pendant une heure, on refroidit à température ambiante puis on neutralise la potasse qui n'a pas réagi par de l'acide méthacrylique (indicateur coloré utilisé : phénolphtaléine). Les cristaux de méthacrylate de potassium formés sont séparés par filtration et séchés sous vide vers 40-60°C au moyen d'un évaporateur rotatif.

Dans un réacteur de 250 ml, muni d'une agitation centrale et d'un réfrigérant, on introduit :

- 68,2 g du méthacrylate de potassium préparé à l'étape précédente et n'ayant subi aucune purification ultérieure,
- 63,3 g de chlorure de benzyle,
- 0,59 g de tétraméthyl éthylènediamine,
- 0,07 g d'ester méthylique d'hydroquinone, et
- 150 ml d'acétonitrile.

Le milieu réactionnel est ensuite porté à la température de 60°C sous bullage d'air sec pendant 6 heures. Après avoir laissé refroidir jusqu'à la température ambiante, on filtre sur verre fritté de porosité 2 et le précipité recueilli est lavé au moyen de 50 ml d'acétonitrile. Le filtrat jaune clair est ensuite strippé au moyen d'un évaporateur rotatif fonctionnant à la température de 60°C et sous une pression de 0,065 bar. L'élimination de l'acétonitrile permet ainsi de récupérer le méthacrylate de benzyle avec le rendement R (exprimé en %) indiqué au tableau ci-après.

EXEMPLE 2

La procédure expérimentale de l'exemple 1 est reproduite à l'exception de la température réactionnelle, qui est fixée à 30°C au lieu de 60°C, et de la quantité de tétraméthyl éthylènediamine, qui est doublée à 1,18 g. Le méthacrylate de benzyle est formé dans ces conditions avec le rendement R indiqué au tableau ci-après.

EXEMPLE 3

On reproduit la procédure expérimentale de l'exemple 1 à la double exception de :

- la température réactionnelle fixée à 50°C, et
- la quantité de tétraméthyl éthylènediamine, portée à 1,18 g.

Le rendement de la réaction effectuée dans ces conditions est indiqué au tableau ci-après.

EXEMPLE 4

On reproduit la procédure expérimentale de l'exemple 1 à l'exception de la quantité de tétraméthyl éthylènediamine, abaissée à 0,295 g. Le rendement de la réaction effectuée dans ces conditions est indiqué au tableau ci-après.

EXEMPLE 5

On reproduit la procédure expérimentale de l'exemple 1 à l'exception de la quantité de méthacrylate de potassium, qui est abaissée à 61,5 g. Le rendement de la réaction effectuée dans ces conditions est indiqué au tableau ci-après.

EXEMPLE 6

On reproduit la procédure expérimentale de l'exemple 1 tout en remplaçant, comme solvant, l'acétonitrile par le toluène en quantité égale et en portant la température réactionnelle à 110°C. Le rendement de la réaction effectuée dans ces conditions est indiqué au tableau ci-après.

TABLEAU

| Exemple | 1 | 2 | 3 | 4 | 5 | 6 |
|---|---|---|---|---|---|---|
| R | 92 | 85 | 91 | 86 | 88 | 83 |

EXEMPLE 7

Dans un réacteur de 2 litres muni d'une agitation mécanique centrale, d'une tête de colonne avec décanteur surmonté d'un réfrigérant et d'une ampoule à brome, on introduit :

- 279 $cm^3$ d'une solution de potasse (concentration 10,5 moles/l) soit une solution contenant 2,95 moles de potasse,
- 1300 grammes de toluène,
- 0,925 gramme de butyl hydroxy toluène.

Le réacteur est ensuite chauffé à une température comprise entre 40-43°C puis on introduit à l'aide d'une ampoule à brome, 250 grammes d'acide méthacrylique en 1 heure 30 minutes.

Le réacteur est ensuite placé sous pression réduite de 200 mbar et chauffé à 60°C : l'eau formée est distillée. On refroidit le milieu réactionnel, puis on le filtre sur Büchner. Les cristaux de méthacrylate de potassium obtenus sont alors séchés sous vide à 50°C au moyen d'un évaporateur rotatif. On obtient le méthacrylate de potassium avec une pureté de 95% (déterminée par analyse RMN).

Dans un réacteur de 250 ml, muni d'une agitation centrale, et d'un réfrigérant, on introduit :

- 71,8 g de méthacrylate de potassium préparé dans l'étape antérieure,
- 80,5 g de chloro-2 chlorure de benzyle,
- 0,845 gramme de tétraméthyl propyl diamine,
- 0,1 gramme de butyl hydroxy toluène,
- 110 grammes de toluène.

Le réacteur est ensuite plongé dans un bain d'huile chauffé à 90°C, puis laissé sous agitation pendant 6 heures et ramené ensuite à la température ambiante au bout de 2 heures : on filtre ensuite sur verre fritté. Le filtrat est ensuite lavé avec 60 $cm^3$ de toluène. On ajoute ensuite 0,158 gramme de l'éther méthylique de l'hydroxyquinone puis on élimine le toluène à l'aide d'un évaporateur rotatif fonctionnant à une température de 60°C sous une pression de 60 mmHg, puis sous une pression de 20 mmHg.

On récupère ainsi le méthacrylate de chloro-2 benzyle avec un rendement de 99,8%.

EXEMPLE 8

L'exemple 7 est répété en mettant en oeuvre les ingrédients suivants :
- 70 grammes de méthacrylate de potassium,
- 97,9 grammes de dichloro-2,4 chlorure de benzyle
- 0,754 gramme de tétraméthyl éthylènediamine,
- 0,1 gramme de butyl hydroxy toluène,
- 110 grammes de toluène.

Après traitement comme dans l'exemple 7, on obtient le méthacrylate de dichloro-2,4 benzyle avec un rendement de 97,6%.

EXEMPLE 9

L'exemple 7 est répété à l'aide des réactifs suivants :
- 70 grammes de méthacrylate de potassium,
- 140,6 grammes de méthyl-4 chlorure de benzyle,
- 0,787 gramme de tétraméthyl éthylènediamine,
- 0,5 gramme de butyl hydroxy toluène,
- 110 grammes de toluène.

On obtient le méthacrylate de méthyl-4 benzyle avec un rendement de 97,4%.

EXEMPLE 10

On reproduit la procédure de l'exemple 7 en mettant en oeuvre les réactifs suivants :
- 70,3 grammes de méthacrylate de potassium,
- 43,8 grammes de chloro méthyl-4 chlorure de benzyle,
- 0,824 gramme de tétraméthyl éthylènediamine,
- 0,10 gramme de butyl hydroxy toluène,
- 170 grammes de toluène.

Après réaction et traitement comme dans l'exemple 1, on obtient le diméthacrylate de benzyle avec un rendement de 68,6%.

EXEMPLE 11

La procédure expérimentale de l'exemple 7 est reproduite à l'aide des réactifs suivants :
- 70,5 grammes de méthacrylate de potassium,
- 81 grammes de chloro-4 chlorure de benzyle,
- 0,733 gramme de tétraméthyl éthylènediamine,
- 0,1 gramme de butyl hydroxy toluène,
- 110 grammes de toluène.

On obtient en fin de réaction, le méthacrylate de chloro-4 benzyle avec un rendement de 98,2%.

EXEMPLE 12

L'exemple 7 est répété en mettant en oeuvre les ingrédients suivants :
- 70 grammes de méthacrylate de potassium,
- 109,9 grammes de vinyl-3 chlorure de benzyle,
- 0,79 gramme de tétraméthyl éthylènediamine,
- 0,1 gramme de butyl hydroxy toluène,
- 110 grammes de toluène.

Après traitement comme dans l'exemple 1, on recueille le méthacrylate de vinyle-3 benzyle.

## Revendications

1. Procédé de fabrication du méthacrylate de benzyle ou d'un de ses dérivés halogénés ou alcoylés sur le noyau aromatique présentant la formule générale :

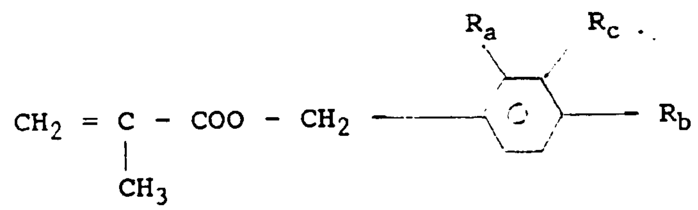

dans laquelle :

- $R_a$ = H ou Cl
- $R_b$ = H, Cl, $CH_3$ ou

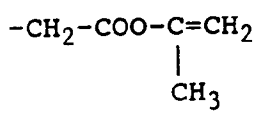

- $R_c$ = H ou $CH_2 = CH$-

selon lequel on fait réagir le chlorure de benzyle ou un de ses dérivés halogénés ou alcoylés sur le noyau aromatique sur un méthacrylate de métal alcalin, caractérisé en ce que la réaction est effectuée en présence d'au plus 3% en moles, par rapport au chlorure de benzyle ou d'un de ses dérivés d'une amine tertiaire.

2. Procédé de fabrication selon la revendication 1, caractérisé en ce que le métal alcalin est choisi parmi lithium, potassium et sodium.

3. Procédé de fabrication selon l'une des revendications 1 et 2, caractérisé en ce que l'amine tertiaire est une diamine tertiaire de formule générale :

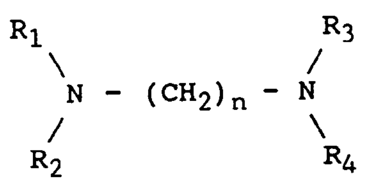

(I)

dans laquelle n est un nombre entier allant de 1 à 10, $R_1$, $R_2$, $R_3$ et $R_4$, identiques ou différents, sont des radicaux allyle, cycloallyle ou aryle ayant de 1 à 12 atomes de carbone, le cas échéant fonctionnalisés et le cas échéant tels qu'ils forment un hétérocycle avec l(es) atome(s) d'azote qui les portent.

4. Procédé de fabrication selon l'une des revendications 1 à 3, caractérisé en ce que l'amine tertiaire est la tétraméthyléthylènediamine et la tétraméthylpropyldiamine.

5. Procédé de fabrication selon l'une des revendications 1 à 4, caractérisé en ce que le rapport molaire méthacrylate de métal alcalin/chlorure de benzyle ou un de ses dérivés est compris entre 0,9 et 1,5.

6. Procédé de fabrication selon l'une des revendications 1 à 5, caractérisé en ce que la proportion molaire amine tertiaire/chlorure de benzyle ou un de ses dérivés est comprise entre 0,5 et 2%.

7. Procédé de fabrication selon l'une des revendications 1 à 6, caractérisé en ce que la réaction est effectuée en présence d'un solvant de très grande polarité.

8. Procédé de fabrication selon l'une des revendications 1 à 7, caractérisé en ce que la réaction est effectuée en présence d'un solvant choisi parmi l'acétonitrile et le toluène.

9. Procédé de fabrication selon l'une des revendications 1 à 8, caractérisé en ce que la réaction est effectuée à une température choisie entre 20°C et 140°C.

**10.** Procédé de fabrication selon l'une des revendications 1 à 9, caractérisé en ce que la réaction est effectuée pendant une durée comprise entre 1 et 10 heures.

## Claims

**1.** Process for the manufacture of benzyl methacrylate or of one of its derivatives which are halogenated or alkylated on the aromatic nucleus, having the general formula:

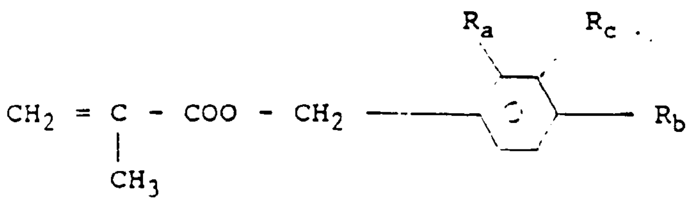

in which:

- $R_a$ = H or Cl
- $R_b$ = H, Cl, $CH_3$ or

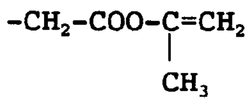

- $R_c$ = H or $CH_2$=CH-

according to which benzyl chloride or one of its derivatives which are halogenated or alkylated on the aromatic nucleus is reacted with an alkali metal methacrylate, characterized in that the reaction is carried out in the presence of not more than 3 mol%, based on the benzyl chloride or one of its derivatives, of a tertiary amine.

**2.** Process of manufacture according to Claim 1, characterized in that the alkali metal is chosen from lithium, potassium and sodium.

**3.** Process of manufacture according to either of Claims 1 and 2, characterized in that the tertiary amine is a tertiary diamine of general formula:

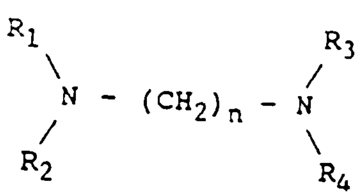

(I)

in which n is an integer ranging from 1 to 10, and $R_1$, $R_2$, $R_3$ and $R_4$, which are identical or different, are alkyl, cycloalkyl or aryl radicals containing from 1 to 12 carbon atoms, functionalized if appropriate and, if appropriate, such that they form a heterocyclic ring with the nitrogen atom(s) carrying them.

**4.** Process of manufacture according to one of Claims 1 to 3, characterized in that the tertiary amine is tetramethylethylenediamine and tetramethylpropyldiamine.

**5.** Process of manufacture according to one of Claims 1 to 4, characterized in that the molar ratio of alkali metal methacrylate/benzyl chloride or one of its derivatives is between 0.9 and 1.5.

**6.** Process of manufacture according to one of Claims 1 to 5, characterized in that the molar proportion of tertiary amine/benzyl chloride or one of its derivatives is between 0.5 and 2 %.

**7.** Process of manufacture according to one of Claims 1 to 6, characterized in that the reaction is carried

out in the presence of a solvent of very high polarity.

8. Process of manufacture according to one of Claims 1 to 7, characterized in that the reaction is carried out in the presence of a solvent chosen from acetonitrile and toluene.

9. Process of manufacture according to one of Claims 1 to 8, characterized in that the reaction is carried out at a temperature chosen between 20°C and 140°C.

10. Process of manufacture according to one of Claims 1 to 9, characterized in that the reaction is carried out for a period of between 1 and 10 hours.

**Patentansprüche**

1. Verfahren zur Herstellung von Benzylmethacrylat oder einem seiner am aromatischen Ring substituierten Halogen- oder Alkylderivate, mit der allgemeinen Formel

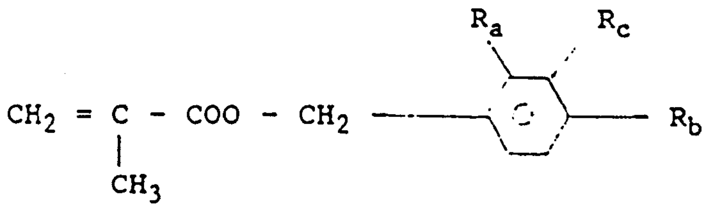

worin:
- $R_a$ = H oder Cl
- $R_b$ = H, Cl, $CH_3$ oder

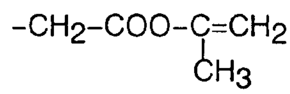

- $R_c$ = H oder $CH_2$ = CH-,

gemäß welchem Verfahren man Benzylchlorid oder eines seiner am aromatischen Ring substituierten Halogen- oder Alkylderivate mit einem Alkalimetallmethacrylat zur Reaktion bringt, dadurch gekennzeichnet, daß man die Reaktion in Gegenwart von höchstens 3 Mol-% eines tertiären Amins, bezogen auf das Benzylchlorid oder eines seiner Derivate, durchführt.

2. Herstellungsverfahren nach Anspruch 1, dadurch gekennzeichnet, daß das Alkalimetall ausgewählt wird aus der Gruppe bestehend aus Lithium, Kalium und Natrium.

3. Herstellungsverfahren nach Anspruch 1 oder 2, dadurch gekennzeichnet, daß das tertiäre Amin ein tertiäres Diamin der allgemeinen Formel:

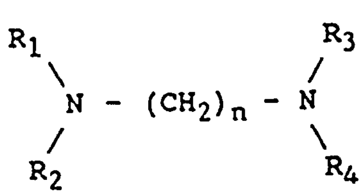

(I)

ist, worin n eine ganze Zahl im Bereich von 1 bis 10 ist und $R_1$, $R_2$, $R_3$ und $R_4$, die identisch oder verschieden sind, Alkyl-Cycloalkyl- oder Arylreste mit 1 bis 12 Kohlenstoffatomen sind und gegebenenfalls funktionalisiert und gegebenenfalls so sind, daß sie mit dem (den) Stickstoffatom(en), das (die) sie tragen, eine heterocyclische Verbindung bilden.

4. Herstellungsverfahren nach einem der Ansprüche 1 bis 3, dadurch gekennzeichnet, daß das tertiäre Amin Tetramethylethylendiamin oder Tetramethylpropyldiamin ist.

5. Herstellungsverfahren nach einem der Ansprüche 1 bis 4, dadurch gekennzeichnet, daß das Molverhältnis zwischen Alkalimetallmethacrylat und Benzylchlorid oder einem seiner Derivate 0,9 bis 1,5 beträgt.

6. Herstellungsverfahren nach einem der Ansprüche 1 bis 5, dadurch gekennzeichnet, daß der Molprozentsatz an tertiärem Amin, bezogen auf das Benzylchlorid oder eines seiner Derivate, 0,5 bis 2% beträgt.

7. Herstellungsverfahren nach einem der Ansprüche 1 bis 6, dadurch gekennzeichnet, daß die Reaktion in Gegenwart eines Lösungsmittels mit sehr hoher Polarität durchgeführt wird.

8. Herstellungsverfahren nach einem der Ansprüche 1 bis 7, dadurch gekennzeichnet, daß die Reaktion in Gegenwart eines Lösungsmittels durchgeführt wird, das ausgewählt ist aus der Gruppe bestehend aus Acetonitril und Toluol.

9. Herstellungsverfahren nach einem der Ansprüche 1 bis 8, dadurch gekennzeichnet, daß die Reaktion bei einer Temperatur von 20° bis 140°C durchgeführt wird.

10. Herstellungsverfahren nach einem der Ansprüche 1 bis 9, dadurch gekennzeichnet, daß die Reaktion für eine Dauer von 1 bis 10 Stunden durchgeführt wird.